# EUROPÄISCHE PATENTANMELDUNG

(11) **EP 0 559 933 A1**
(43) Veröffentlichungstag der Anmeldung: **15.09.1993**
(21) Anmeldenummer: 92104098.6
(22) Anmeldetag: 10.03.1992
(51) Int. Cl.: A61N 1/05, A61N 1/39

(54) **Elektrodenanordnung für einen implantierbaren Defibrillator/Kardiovertierer**

(71) Anmelder: Pacesetter AB, S-171 95 Solna (SE)
(72) Erfinder: Alm, Malin, S-171 49 Solna (SE); Hirschberg, Jakub, S-183 44 Täby (SE); Peterson, Lars Olof, S-161 37 Bromma (SE); Stegfeldt, Olof, S-138 00 Älta (SE); Bowald, Staffan, S-749 10 Almunge (SE)
(74) Vertreter: Lettström, Richard Wilhelm

(57) **Zusammenfassung**

Eine Elektrodenanordnung für Defibrillierungszwecke weist zwei intravaskuläre Elektroden und eine Flächenelektrode (13) außerhalb des Herzens auf. Die intravaskulären Elektroden (9,10) sind beziehungsweise in der vena cava inferior (8) und in der vena cava superior (11) angeordnet.

## Beschreibung

Die Erfindung betrifft eine Elektrodenanordnung für einen implantierbaren Defibrillator/Kardiovertierer, der einen ausgangsseitig mit der Elektrodenanordnung verbundenen Impulsgenerator zur Erzeugung von elektrischen Defibrillierungs-/Kardiovertierungsimpulsen aufweist, mit zwei intravaskulären Elektroden, von denen eine im Bereich der vena cava superior angeordnet ist, und mit einer dritten Flächenelektrode außerhalb des Herzens im Bereich des linken Ventrikels.

Bei einer derartigen, aus der US-A-4 662 377 bekannten Elektrodenanordnung, sind an einem in die rechte Herzhälfte einzuführenden Katheter zwei Elektroden derart voneinander beabstandet angeordnet, daß die eine Elektrode im rechten Ventrikel des Herzens und die andere Elektrode in der vena cava superior zu liegen kommt. Außerhalb des Herzens ist eine dem linken Ventrikel gegenüberliegende Flächenelektrode subkutan angeordnet. Die Flächenelektrode ist mit der Elektrode in der vena cava superior verbunden und an einem Ausgangsanschluß eines implantierbaren Defibrillators angeschlossen, dessen anderer Ausgangsanschluß mit der Elektrode im Ventrikel verbunden ist. Der Katheter weist an seinem distalen Ende eine zusätzliche Herzschrittmacherelektrode auf, die zusammen mit der Elektrode im Ventrikel zum Detektieren und Stimulieren von Herzereignissen dient.

Wie aus der US-A-5 044 375 hervorgeht, können für die Elektroden zum Defibrillieren des Herzens und die Elektroden zum Detektieren von Herzereignissen jeweils unterschiedliche Katheter vorgesehen werden, wobei jedoch beim Implantieren der Katheter im Herzen darauf zu achten ist, daß keine Kurzschlüsse zwischen den Defibrillierungselektroden und den Detektorelektroden entstehen können.

Aus der US-A-4 708 145 ist eine weitere Elektrodenanordnung für einen Defibrillator/Kardiovertierer bekannt, bei der ebenfalls eine Elektrode im rechten Ventrikel und eine andere Elektrode, die in der vena cava superior positioniert ist, an einem gemeinsamen Katheter angeordnet sind und eine Flächenelektrode vorgesehen ist, die sowohl subkutan als auch epikardiell oder in der Nähe des Zwerchfells angeordnet werden kann. Die Abgabe von Defibrillierungsimpulsen erfolgt sequentiell zwischen der Elektrode in der vena cava superior und der Elektrode im Ventrikel einerseits und der Flächenelektrode und der Elektrode im Ventrikel andererseits. Anstelle in der vena cava superior kann die entsprechende Elektrode auch in der vena cava inferior angeordnet sein. Es ist ferner eine Elektrodenanordnung angegeben, bei der ausschließlich Flächenelektroden auf dem Herzen angeordnet sind.

Schließlich ist aus der EP-A-0 373 953 eine Elektrodenanordnung zum Defibrillieren eines Herzens bekannt, bei der im Falle der Verwendung von drei Elektroden eine Elektrode über einen Katheter im rechten Ventrikel, eine weitere Elektrode über einen weiteren Katheter in der vena cordis magna und eine dritte Flächenelektrode subkutan dem linken Ventrikel gegenüberliegend angeordnet ist. Alternativ ist vorgesehen, daß die Defibrillation zwischen nur jeweils zwei der oben genannten Elektroden erfolgt.

Bei Verwendung von mehr als zwei Elektroden besteht jedoch der Vorteil, daß im Falle der Defibrillation des Herzens eine bessere Verteilung der Stromdichte auf unterschiedliche Zonen des Herzmuskels möglich ist. Die obengenannten bekannten Elektrodenanordnungen mit mehr als zwei Elektroden weisen alle jeweils einen endokardiellen Katheter mit einer ventrikulären Elektrode auf. Wegen der Größe der Defibrillierungselektroden, der erhöhten Anforderungen an die elektrische Isolierung des Katheters außerhalb der Elektroden und der im Vergleich zu Herzschrittmacherelektroden dickeren Elektrodenzuleitungen, sind jedoch endokardielle Katheter für Defibrillierungszwecke relativ dick und steif. Dies kann zu einer unnötigen mechanischen Reizung des Herzens führen; die Atrioventrikularklappen können nicht richtig schließen und es können sich vermehrt Klümpchen im Blut bilden. Es ist zwar denkbar, wie aus der obengenannten US-A-4 708 145 bekannt, ausschließlich Flächenelektroden außerhalb des Herzens vorzusehen, allerdings müssen diese unter Öffnung des Brustkorbes direkt auf dem Herzen appliziert werden, wenn die Defibrillierungsenergie optimal ausgenutzt werden soll.

Der Erfindung liegt die Aufgabe zugrunde, eine Elektrodenanordnung zur Defibrillierung eines Herzens anzugeben, die eine hohe Ausnutzung der Defibrillierungsenergie ermöglicht, ohne daß eine endokardielle Defibrillierungselektrode erforderlich ist.

Gemäß der Erfindung wird die Aufgabe dadurch gelöst, daß bei der Elektrodenanordnung der eingangs angegebenen Art die andere intravaskuläre Elektrode im Bereich der vena cava inferior angeordnet ist. Auf diese Weise wird erreicht, daß zwei Defibrillierungselektroden, nämlich die in der vena cava superior und die in der vena cava inferior in unmittelbarer Nähe zum Herzen angeordnet sind und zusammen mit der Flächenelektrode eine die Defibrillierungsenergie optimal zur Defibrillierung des Herzens ausnutzende Elektrodenanordnung bilden, ohne daß hierzu eine endokardielle Elektrode erforderlich ist. Hierdurch wird in vorteilhafter Weise eine mechanische Reizung des Herzens und die Bildung von Blutklümpchen innerhalb des Herzens verhindert; eine Perforierung der Atrioventrikularklappen findet nicht statt und mögliche Beschädigungen des Atrioventrikularknotens sind ausgeschlossen. Insbesondere bei infarktgeschädigten Patienten liegt oft eine Überempfindlichkeit des Herzens gegenüber mechanischen Reizungen vor, weswegen die erfindungsgemäße Elektrodenanordnung bei solchen Patienten von besonderem Vorteil ist.

Unter Ausnutzung des Vorteils, daß sich bei der erfindungsgemäßen Elektrodenanordnung keine Defibrillierungselektrode im Inneren des Herzens befindet, kann im Rahmen der Erfindung mindestens eine endokardielle Elektrode vorgesehen sein, die über einen eigenen flexiblen Elektrodenkatheter mit einer Detektor- und/oder Herzschrittmacherschaltung des Defibrillators/Kardiovertierers verbunden ist. Da diese Elektrode nicht zur Übertragung von Hochspannungsimpulsen dient, kann der entsprechende Elektrodenkatheter entsprechend dünn und flexibel ausgebildet sein, so daß die oben in Verbindung mit einem Katheter für Defibrillierungszwecke angegebenen Nachteile nicht oder in nur geringem Maße zutreffen. Ein weiterer Vorteil besteht darin, daß die Detektor- bzw. Stimulationselektrode im Herzen räumlich vollständig von den Defibrillierungselektroden getrennt angeordnet ist, so daß die Gefahr von Kurzschlüssen zwischen der endokardiellen Elektrode und den Defibrillierungselektroden völlig ausgeschlossen ist. Dementsprechend brauchen auch keine besonderen Maßnahmen zur Isolierung der Elektroden voneinander getroffen werden. Als indifferente Gegenelektrode zu der Detektor- bzw. Stimulationselektrode kann eine weitere Ringelektrode an dem Elektrodenkatheter vorgesehen sein. Es können alternativ dazu auch das Gehäuse des Defibrillators/Kardiovertierers, die Flächenelektrode oder die beiden intravaskulären Defibrillierungselektroden, einzeln oder gemeinsam als Gegenelektrode dienen.

Aufgrund ihrer Lage in der vena cava superior und in der vena cava inferior sind die beiden intravaskulären Defibrillierungselektroden vorzugsweise voneinander beabstandet an einem gemeinsamen Katheter angeordnet. Dadurch wird erreicht, daß der Abstand zwischen den beiden Elektroden genau definiert ist und daß sich beide Elektroden gleichzeitig an den für sie vorgesehenen Stellen positionieren lassen. Dabei ist vorzugsweise die Elektrode im Bereich der vena cava inferior als distale Elektrode an dem Katheter ausgebildet, wobei die Positionierung der Elektrodenanordnung von der vena cava superior her erfolgt.

Um eine stabile Lage des Katheters in seiner implantierten Endstellung zu erreichen, weist der Katheter an seinem distalen Endbereich vorzugsweise Mittel zur Fixierung in der vena cava inferior auf. Diese Mittel können beispielsweise in Form von Spreizelementen oder durch eine schraubenfederartige Ausbildung der Elektrode in der vena cava inferior realisiert werden, wodurch das distale Ende des Katheters in der Vene festgeklemmt wird. Eine weitere Möglichkeit besteht darin, daß das distale Ende mit Hilfe von Haken oder schraubenlinienförmigen Fortsätzen in der Venenwand verankerbar ist.

Entsprechend einer bevorzugten Ausbildung der erfindungsgemäßen Elektrodenanordnung weist der Katheter im Bereich zwischen den beiden intravaskulären Elektroden Mittel zur Fixierung des Katheters im Bereich des rechten Atriums auf. Dadurch wird verhindert, daß der Katheter in den relativ großräumigen Venenbereichen seine Lage verändern kann und unter Umständen in das Innere des Herzens geraten kann. Die Mittel zum Fixien des Katheters im Bereich des rechten Atriums bestehen vorzugsweise aus einer von einem geradlinigen Verlauf abweichenden Verformung des Katheters oder aus seitlich vom Katheter abstehenden Spreizelementen. Dabei ist vorgesehen, daß diese Fixierungsmittel erst dann aktiviert werden, wenn der Katheter in seiner endgültigen Lage positioniert worden ist. Die von dem gradlinigen Verlauf abweichende Verformung des Katheters kann dabei in der Weise bewerkstelligt werden, daß der Katheter an der entsprechenden Stelle vorgebeugt ist und nach Entfernen eines im Inneren des Katheters geführten Steuerdrahtes die vorgesehene Verformung annimmt. Eine weitere Möglichkeit zur Verformung des Katheters besteht darin, daß dieser Elemente aus einer Metallegierung enthält, die beim Erreichen eines vorgegebenen, hier vorzugsweise der Körpertemperatur entsprechenden Temperaturniveaus eine vorgegebene Form annehmen.

Entsprechend einer vorteilhaften Weiterbildung der erfindungsgemäßen Elektrodenanordnung, die eine Mehrfachausnutzung des Katheters ermöglicht, ragen die Mittel zum Fixieren des Katheters in den Bereich des rechten Atriums hinein und weisen dort eine atrielle Elektrode auf, die über eine innerhalb des Katheters geführte Elektrodenleitung mit einer Detektor- und/oder Herzschrittmacherschaltung des Defibrillators/Kardiovertierers verbunden ist.

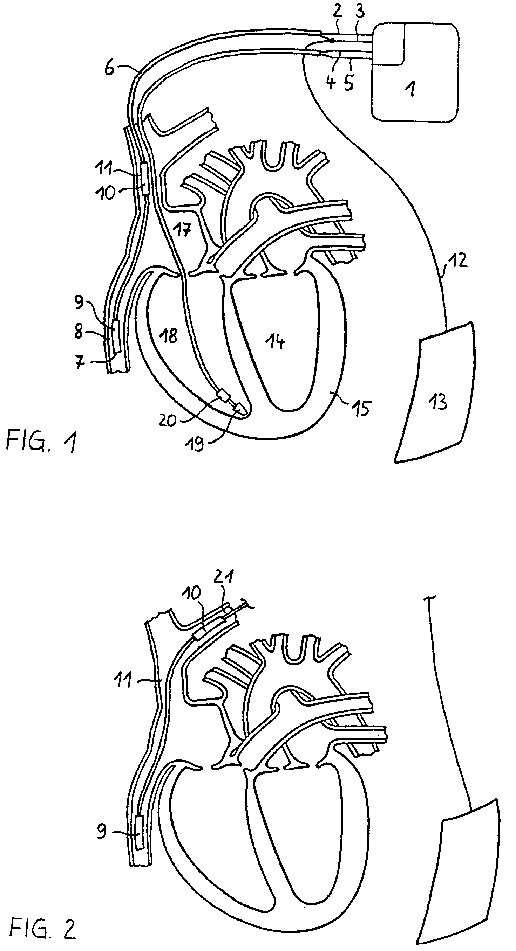

Zur Erläuterung der Erfindung wird im folgenden auf die Figuren der Zeichnung Bezug genommen, wobei die Figuren 1 bis 6 unterschiedliche Ausführungsbeispiele der erfindungsgemäßen Elektrodenanordnung zeigen.

Figur 1 zeigt einen implantierbaren Defibrillator/ Kardiovertierer 1 mit zwei Ausgangsanschlüssen 2 und 3 zur Abgabe von Defibrillierungsimpulsen und zwei weiteren Anschlüssen 4 und 5, die mit einer hier nicht gezeigten Detektor- und Herzschrittmacherschaltung im Inneren des Defibrillators/Kardiovertierers 1 verbunden sind. An den Ausgangsanschlüssen 2 und 3 ist ein Katheter 6 angeschlossen, der an seinem distalen Ende 7 eine in der vena cava inferior 8 positionierte Defibrillierungselektrode 9 aufweist und von dieser beabstandet eine weitere Defibrillierungselektrode 10 aufweist, die in der vena cava superior 11 angeordnet ist. Bei dem gezeigten Ausführungsbeispiel ist die Elektrode 9 mit dem Ausgangsanschluß 2 und die Elektrode 10 mit dem Ausgangsanschluß 3 verbunden, an dem über eine isolierte Leitung 12 eine Flächenelektrode 13 angeschlossen ist, die im Bereich des linken Ventrikels 14 des Herzens 15 an diesem oder im Abstand zum Herzen 15 subkutan implantiert ist. An den Anschlüssen 4 und 5 ist ein Elektrodenkatheter 16 angeschlossen, der durch die vena cava superior 11 und das rechte Atrium 17 des Herzens 15 hindurch in den rechten Ventrikel 18 geführt ist und dort an seinem distalen Ende eine Spitzenelektrode 19 und eine von dieser in proximaler Richtung beabstandete Ringelektrode 20 aufweist.

Bei dem in Figur 1 gezeigten Ausführungsbeispiel erfolgt die Defibrillation des Herzens 15 zwischen der Elektrode 9 in der vena cava inferior einerseits und den miteinander verbundenen Elektroden 10 und 13 in der vena cava superior beziehungsweise außerhalb des Herzens 15 andererseits. Es ist aber auch möglich, daß die Defibrillation zwischen einer anderen der genannten Defibrillierungselektroden 9, 10 und 13 und den beiden übrigen Defibrillierungselektroden erfolgt. Ferner ist es möglich, daß die Elektroden 9, 10 und 13 paarweise nacheinander mit einem Defibrillierungsimpuls beaufschlagt werden oder, daß die drei Defibrillierungselektroden 9, 10 und 13 gleichzeitig mit unterschiedlichen Spannungen beaufschlagt werden. Es können mono-, bi- oder mehrphasische Defibrillationsimpulse vorgesehen werden.

Figur 2 zeigt eine weitere Möglichkeit zur Positionierung des Katheters 6 mit den Defibrillierungselektroden 9 und 10 wobei der Katheter 6 durch die vena brachiocephalica 21 in die vena cava geführt ist und die mit 10 bezeichnete Elektrode in den Bereich des Übergangs von der vena axillaris 21 in die vena cava superior 11 zu liegen kommt.

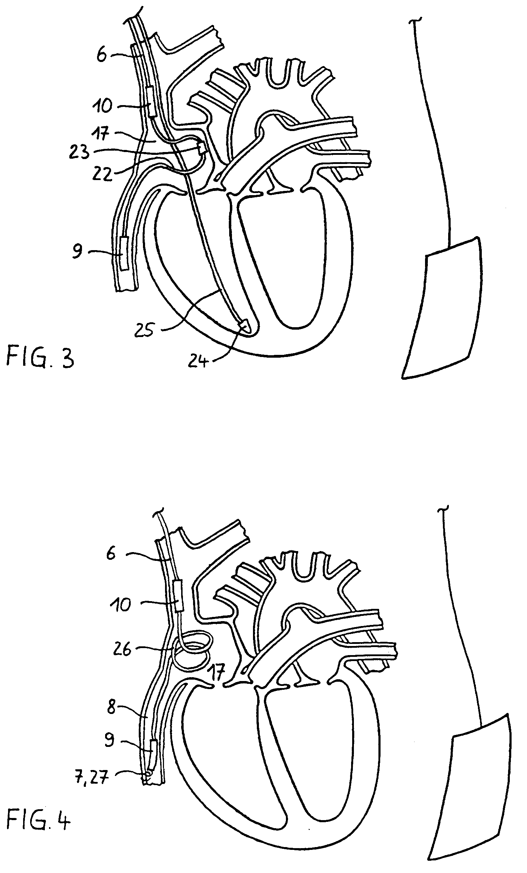

Bei dem in Figur 3 gezeigten Ausführungsbeispiel weist der Katheter 6 im Bereich zwischen den beiden intravaskulären Elektroden 9 und 10 Mittel bestehend aus einer von dem geradlinigen Verlauf abweichenden Verformung 22 des Katheters 6 auf, wobei die Verformung 22 des Katheters 6 in das Atrium 17 hineinreicht und dort eine atrielle Elektrode 23 aufweist, die über eine innerhalb des Katheters 6 geführte, hier nicht gezeigte Elektrodenleitung mit der Detektor- und/oder Herzschrittmacherschaltung des Defibrillators/Kardiovertierers 1 (Fig. 1) verbunden ist. Der atriellen Elektrode 23 ist eine ventrikuläre Elektrode 24 zugeordnet, die über einen eigenen Elektrodenkatheter 25 mit der Detektor- und/oder Herzschrittmacherschaltung des Defibrillators/Kardiovertierers 1 (Fig. 1) verbunden ist. Anstelle der atriellen Elektrode ist auch eine Meßsonde für Drucke, Strömung, Temperatur oder Gassättigung des venösen Bluts denkbar.

Bei dem in Figur 4 gezeigen Ausführungsbeispiel der erfindungsgemäßen Elektrodenanordnung weist der Katheter 6 im Bereich zwischen den beiden intravaskulären Elektroden 9 und 10 eine spiralförmige Verformung 26 auf, die den Katheter 6 im Bereich des rechten Atriums 17 fixiert. Zusätzlich ist der Katheter 6 an seinem distalen Ende 7 mit einer schraubenförmigen Spitze 27 versehen, die den Katheter 6 in der Wand der vena cava inferior 8 fixiert.

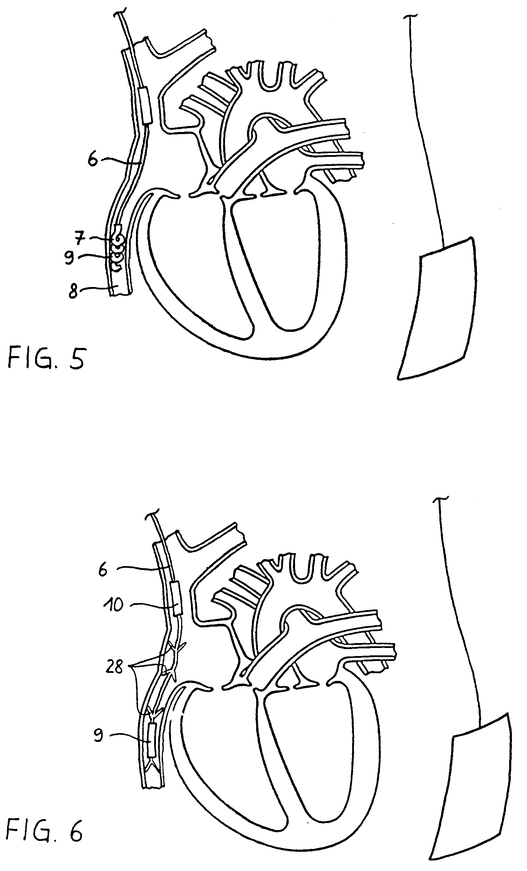

Bei dem in Figur 5 gezeigten Ausführungsbeispiel der erfindungsgemäßen Elektrodenanordnung ist die Elektrode 9 am distalen Ende 7 des Katheters 6 schraubenlinienförmig ausgebildet und auf diese Weise in der vena cava inferior 8 fixiert.

Figur 6 zeigt schließlich ein Ausführungsbeispiel der erfindungsgemäßen Elektrodenanordnung, bei dem der Katheter 6 im Bereich zwischen den beiden intravaskulären Elektroden 9 und 10 sowie im Bereich der Elektrode 9 Spreizelemente 28 zur Fixierung des Katheters 6 aufweist.

### Bezugszeichenliste

- 1: Defibrillator/Kardiovertierer
- 2,3: Ausgangsanschlüsse von 1
- 4,5: weitere Anschlüsse von 1
- 6: Katheter
- 7: distales Ende von 6
- 8: Defibrillierungselektroden in 9
- 9: vena cava inferior
- 10: Defibrilllierungselektrode in 11
- 11: vena cava superior
- 12: Leitung
- 13: Flächenelektrode
- 14: linker Ventrikel
- 15: Herz
- 16: Elektrodenkatheter
- 17: rechtes Atrium
- 18: rechter Ventrikel
- 19: Spitzenelektrode
- 20: Ringelektrode
- 21: vena brachiocephalica
- 22: Verformung von 6
- 23: atrielle Elektrode
- 24: ventrikuläre Elektrode
- 25: Elektrodenkatheter
- 26: Verformung von 6
- 27: schraubenförmige Spitze
- 28: Spreizelemente

## Patentansprüche

1. Elektrodenanordnung für einen implantierbaren Defibrillator/Kardiovertierer (1), der einen ausgangsseitig mit der Elektrodenanordnung verbundene Impulsgenerator zur Erzeugung von elektrischen Defibrillierungs-/Kardiovertierungsimpulsen aufweist, mit zwei intravaskulären Elektroden (9, 10), von denen eine (10) im Bereich der vena cava superior (11) angeordnet ist, und mit einer dritten Flächenelektrode (13) außerhalb des Herzens (15) im Bereich des linken Ventrikels (14), **dadurch gekennzeichnet**, daß die andere intravaskuläre Elektrode (9) im Bereich der vena cava inferior (8) angeordnet ist.

2. Elektrodenanordnung nach Anspruch 1, **dadurch gekennzeichnet,** daß mindestens eine endokardielle Elektrode (19, 20; 24) vorgesehen ist, die über einen eigenen flexiblen Elektrodenkatheter (16; 25) mit einer Detektor- und/oder Herzschrittmacherschaltung des Defibrillators/Kardiovertierers (1) verbunden ist.

3. Elektrodenanordnung nach Anspruch 1 oder 2**, dadurch gekennzeichnet,** daß die beiden intravaskulären Elektroden (9, 10) voneinander beabstandet an einem gemeinsamen Katheter (6) angeordnet sind.

4. Elektrodenanordnung nach Anspruch 3, **dadurch gekennzeichnet,** daß die Elektrode (9) im Bereich der vena cava inferior (8) als distale Elektrode an dem Katheter (6) ausgebildet ist.

5. Elektrodenanordnung nach Anspruch 4, **dadurch gekennzeichnet,** daß der Katheter (6) an seinem distalen Endbereich (7) Mittel (27) zur Fixierung in der vena cava inferior (8) aufweist.

6. Elektrodenanordnung nach einem der Ansprüche 3 bis 5, **dadurch gekennzeichnet,** daß der Katheter (6) im Bereich zwischen den beiden intravaskulären Elektroden (9, 10) Mittel (22, 26, 28) zur Fixierung des Katheters (6) im Bereich des rechten Atriums (17) aufweist.

7. Elektrodenanordnung nach Anspruch 6, **dadurch gekennzeichnet,** daß die Mittel zum Fixieren des Katheters (6) im Bereich des rechten Atriums (17) aus einer von einem geradlinigen Verlauf abweichenden Verformung (22, 26) des Katheters (6) bestehen.

8. Elektrodenanordnung nach Anspruch 6, **dadurch gekennzeichnet,** daß die Mittel zum Fixieren des Katheters (6) im Bereich des rechten Atriums (17) aus seitlich von dem Katheter (6) abstehenden Spreizelementen (28) bestehen.

9. Elektrodenanordnung nach einem der Ansprüche 6 bis 8, **dadurch gekennzeichnet,** daß die Mittel (22) zum Fixieren des Katheters (6) im Bereich des rechten Atriums (17) in dieses hineinragen und eine atrielle Elektrode (23) aufweisen, die über eine innerhalb des Katheters (6) geführte Elektrodenleitung mit einer Detektor- und/oder Herzschrittmacherschaltung des Defibrillators/Kardiovertierers (1) verbunden ist.
